# EUROPEAN PATENT SPECIFICATION

(11) **EP 2 663 226 B1**
(45) Date of publication and mention of the grant of the patent: **27.10.2021**
(21) Application number: 11813445.1
(22) Date of filing: 29.12.2011
(51) Int. Cl.: A61B 5/0205, A61B 5/024, A61B 5/11

(54) **ACTIVITY VISUALIZATION DEVICE**
VORRICHTUNG FÜR AKTIVITÄTSVISUALISIERUNG
DISPOSITIF DE VISUALISATION DE L'ACTIVITÉ

(30) Priority: 14.01.2011 EP 11305041
(43) Date of publication of application: 20.11.2013
(73) Proprietor: Koninklijke Philips N.V., 5656 AG Eindhoven (NL); Philips Intellectual Property & Standards GmbH, 20099 Hamburg (DE)
(72) Inventor: BEREZHNYY, Igor, NL-5656 AE Eindhoven (NL); MAASS, Henning, NL-5656 AE Eindhoven (NL); HAAKMA, Reinder, NL-5656 AE Eindhoven (NL); TATOUSEK, Jan, NL-5656 AE Eindhoven (NL)
(74) Representative: Philips Intellectual Property & Standards
(86) International application number: PCT/IB2011/055999
(87) International publication number: WO 2012/095712

(56) References cited:
- WO-A1-97/21983
- WO-A1-2009/053115
- WO-A2-02/05702
- WO-A2-2007/107900
- US-A1- 2008 146 890

## Description

### FIELD OF THE INVENTION

The invention relates to an activity visualization device, more particularly to an activity visualization device for personal use which is adapted to be used in close physical contact with an individual. The invention relates further to an activity visualization method.

### BACKGROUND OF THE INVENTION

In modern life, maintaining a certain level of activity and regularity of lifestyle is important. One of the major obstacles to achieve this goal is the absence of an effective solution for keeping individuals motivated to do so.

At present there are lifestyle monitoring and coaching solutions on the market such as the Philips DirectLife system, which monitor physical activity, and provide the monitoring results and the motivational instructions on a web-based platform.

Exposure to environmental light is a key mechanism that ensures a proper synchronization of the body clock with the solar day cycle. Timing, duration, intensity and spectral composition of exposure to environmental light all have impact on the so-called "entrainment" of a person to a 24-hour circadian rhythm. Further developed systems like the Philips Actiwatch system thus measure activity and light exposure with a device similar to a wrist watch.

In all cases, however, the said devices do not enable the individual to receive a direct feedback with respect to his personal physical activity. Therefore, an individual's motivation to monitor, and, if necessary, amend or modify, his lifestyle (in terms of physical activity) is not improved by said systems.

United States Patent Application Publication No. US 2008/0146890 A1 teaches a wearable apparatus for monitoring physiological and environmental factors. The real-time, noninvasive health and environmental monitors include a plurality of compact sensors integrated within small, low-profile devices, such as earpiece modules. The physiological and environmental data are collected and wirelessly transmitted into a wireless network, where the data are stored and/or processed.

International Patent Application Publication Number WO 97/21983 A1 discloses a system and a method for measuring movement of objects. A device that measures the distance traveled, speed, and height jumped of a person while running or walking is disclosed. Accelerometers and rotational sensors are placed in the sole of one shoe along with an electronic circuit that performs mathematical calculations to determine the distance and height of each step. A radio frequency transmitter sends the distance and height information to a wristwatch or other central receiving unit. A radio frequency receiver in the wristwatch or other unit is coupled to a microprocessor that calculates an output speed based upon step-distance and elapsed time, the distance traveled of the runner from the sum of all previous step distances. The output of the microprocessor is coupled to a display that shows the distance traveled, speed, or height jumped by the runner or walker.

International Patent Application Publication Number WO 2007/107900 A2 teaches a wearable electronic device such as a wrist watch that is supplied with conventional clock with two pointers. The device displays a parameter of how "cool" the wearer has been over the past period as a function of time, using the time axis of one of the pointers. "Coolness" of the wearer is understood as being the ability to cope with stress, and can be based on the measurement of related physiological parameters like heart-rate, body temperature, movement, skin resistance or muscle activity. All physiological parameters can be measured by sensors in the watch or in the strap.

### SUMMARY OF THE INVENTION

It is an object of the present invention to provide a lifestyle monitoring device which improves an individual's motivation to monitor, and, if necessary, amend or modify, his lifestyle.

It is another object of the present invention to provide a lifestyle monitoring device which enables an individual to amend his lifestyle in response to certain conditions.

It is another object of the present invention to provide a lifestyle monitoring device which enables an individual to amend his lifestyle in order to avoid the development of pathologic conditions, or diseases.

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

In a first aspect of the present invention, an activity visualization device for personal use is provided as defined in claim 1.

An accelerometer is a device that measures proper acceleration of a given device equipped with the latter. Such acceleration can for example be effected by shaking the device. Acceleration is quantified as m s⁻², or, popularly, in terms of g-force (g).

As mentioned, the said accelerometer serves to determine the physical activity of the individual. The measured data are logged in a memory over time, and can optionally be processed. On the visualization display the measured and/or logged data are then displayed. Alternatively, or in addition to the former, data resulting from the computational operation thereof can be displayed.

By visualizing the data related to physical activity over time, the individual receives a direct feedback with respect to his personal physical activity in the past, e.g., in the last few days. The inventors have surprisingly found that such device therefore significantly improves an individual's motivation to monitor, and, if necessary, amend or modify, his lifestyle, e.g., in terms of physical activity, and thus directly affect his quality of life.

The computational operations carried out by the processing unit are, for example, statistic operations (e.g., mean activity, standard deviation, normalization, determination of maximum and minimum), or other operations like 1^{st} or higher derivative, 1^{st} or higher integral, etc.

According to the present invention, said device adopts the shape of a watch, preferably a wrist watch. The said shape has a high acceptance among consumers, is lightweight and easy to wear even over extended periods of time, which is important with respect to the present invention because physical activity data are meant to be logged over time. Recent progress in microelectronic allows the implementation of a large number of functions in a device having such shape.

According to the present invention, said device display is adapted in such way that it comprises a dial covering a given period of time, wherein physical activity data or data resulting from the computational operation thereof are displayed as, e.g., a graph, or curve, over time. Preferably, the dial covers a period of time selected from the group consisting of 12 hrs, 24 hrs and/or 1 week.

However, if the dial covers a given period of time, this does not mean that the capability of the device to display data related to physical activity is restricted to said period. By choosing different display options, a plurality of such periods can be covered. In this case, the most recent data set can be displayed with highest colour saturation, while data sets of earlier periods (e.g., of the days before) are displayed with decreasing colour saturation. In such way, overlays are created, in which the graphs representing the physical activity of an earlier period seem to slowly "fade away".

Thus, it is possible for the individual to see trend changes, e.g., in his physical activity, over time, and in a time-resolved fashion. The individual can, e.g., compare the physical activity during his usual workout time from day to day.

Furthermore, it is preferred that said dial is a circular dial. In this embodiment, a circular section of 360 ° can be used to display a given period of time, e.g., 12 hrs, 24 hrs or 1 week. Due to the circular nature of the dial the line graphs, which display data related to physical activity over time, may adopt a circular or curved shape.

As mentioned earlier, a plurality of such periods can be covered by using different colour saturations for each period, e.g., for each day. The circular nature of the graphs representing the physical activity of different time periods (e.g., several consecutive days) reflects the periodically repeating sequence of days, and can thus be received intuitively by the individual.

In yet another preferred aspect of the invention, said device further comprises at least one sensor for measuring physiological data of the individual, said sensor being selected from the group consisting of:
- heart rate sensor
- breathing rate sensor, and/or
- body or skin temperature sensor.

Like the accelerometer, the respective sensors can also be used to determine physical activity of the individual, either alone or in combination with one another, or with the accelerometer. The respective data are logged over time by the memory, too and measured and/or logged data can as well be subject to computational operations. Further, measured and/or logged data, or data resulting from the computational operation thereof, can as well be presented in the visualization display.

The device further comprises at least one sensor for measuring environmental data, said sensor being selected from the group consisting of
- environmental temperature sensor,
- environmental air humidity sensor,
- environmental atmospheric pressure sensor,
- environmental ozone concentration sensor,
- environmental noise sensor,
- environmental light intensity sensor, and/or
- environmental light spectrometer.

Among these options, an environmental light intensity sensor, and an environmental light spectrometer are particularly preferred. As mentioned above already, exposure to environmental light is a key mechanism that ensures a proper synchronization of the body clock with the solar day cycle. Timing, duration, intensity and spectral composition of exposure to environmental light all have impact on the so-called "entrainment" of a person to a 24-hour circadian rhythm. Similar findings apply to the spectral composition of the environmental light an individual is exposed to. Warm light, (e.g., with a high long-wave content), has a comforting effect, while cold light, (e.g., with a high short-wave content), has an alerting effect.

The implementation of an environmental light intensity sensor, and/or an environmental light spectrometer are particularly preferred is a prerequisite to log the exposure of an individual to environmental light over time. The measured and/or logged data can be subject to computational operations. Furthermore, the measured and/or logged data, or data resulting from the computational operation thereof, can be presented in the visualization display, in order to give a feedback, to the individual, about circadian light exposure patterns.

It has for example been shown that restorative sleep can only occur in synchrony with the body clock. For certain people who have a phase shift of their internal body clock relative to the social schedules around them, exposure to bright light at well-defined times can be used to shift their body clock forwards or backwards to better align it with social requirements.

In order to prevent sleep problems caused from excessive exposure to bright light in the evening, it is furthermore recommended to follow certain sleep hygiene rules which have been developed by sleep clinicians, and under which exposure to bright light is to be avoided at certain times.

Further, for the treatment of seasonal affective disorder (winter depression), timed, regular exposure to bright light is an effective means.

Similar findings as above apply to environmental temperature, environmental air humidity, environmental atmospheric pressure and environmental ozone concentration. All these factors have an influence on the individual's physiology and well-being. Therefore, the measured and/or logged temperature data, air humidity data, atmospheric pressure data and/or environmental ozone concentration data, or data resulting from the computational operation thereof, can be presented in the visualization display, in order to give a feedback, to the individual, about the respective parameters.

According to the invention, said device further comprises a communication means for uploading environmental data from an external source, said data selected from the group consisting of:
- environmental temperature,
- environmental air humidity,
- environmental atmospheric pressure,
- environmental ozone concentration,
- sky cloudiness,
- sunrise and/or sunset data,
- environmental light intensity, and/or
- environmental light spectrum. Said external source is preferably a personal computer with an internet connection.

In principle, the same findings apply as above, i.e., the said factors have an influence on the individual's physiology and well-being. Therefore, the uploaded data, or data resulting from the computational operation thereof, can be presented in the visualization display, in order to give a feedback, to the individual, about the respective parameters.

The said communication means can be any suitable communication means which the skilled person deems adequate for the said purposes. Preferably, said communication means is implemented under at least one of the standards selected from the group consisting of WiFi, Bluetooth, UMTS, and/or USB, ZigBee, Near Field Communication and infrared.

The device further comprises an analysis unit which calculates recommendations for said individual with respect to physical activity and/or environmental light exposure. These recommendations can be presented in the visualization display in a user-friendly, intuitive form. It is important to mention that, as mentioned above, not only data obtained from the accelerometer can be used for determining the physical activity, but also data obtained from a heart rate sensor, from a breathing rate sensor, and/or from a body or skin temperature sensor can be used, if one of these sensors is implemented.

Further, it is important that for the respective recommendations, other data can also be taken into account, like environmental temperature, environmental air humidity, environmental atmospheric pressure, environmental ozone concentration, or body or skin temperature. All these factors affect the physiology of the individual particularly with respect to physical activity.

This embodiment further helps to improve an individual's motivation to monitor, and, if necessary, amend or modify, his lifestyle, e.g., in terms of physical activity or environmental light exposure, and thus directly affect his quality of life

Motivating people to seek or avoid bright light exposure at certain times of the day can for example help them to a) self-manage their circadian rhythm to improve sleep, b) follow sleep hygiene rules, or c) counteract winter depression.

According to still another preferred aspect of the invention, said device further comprises a means for indicating the actual time and/or date. Such means is, for example, a single hand-arrow (in case the device has a circular 24 hrs dial). Alternatively, such means comprises two hand arrows, similar to a conventional analog watch. Both can either be accomplished has hardware hand arrows, or displayed on the visualization device, e.g., with a matrix dot display. Alternatively, such means can comprise a digital time display (00:00 type display), e.g., in form of an LCD or LED display, or displayed on the visualization device, e.g., with a matrix dot display. Other embodiments which are suitable for indicating the actual time and/or date can be used as well.

The device according to the present invention finds primarily use as a physical activity monitor which provides a direct feedback with respect to an individual's personal physical activity. Therefore, it increases the individual's motivation to monitor, and, if necessary, amend or modify, his lifestyle (in terms of physical activity), and can thus contribute to an enhancement of life quality. For this means, a 24 hrs time display is preferably used. The device may furthermore serve to display personally recommended levels of physical activity, to display personally recommended sleeping times, to display times when exposure to light should be avoided or sought and/or to display times when food intake should be avoided, or carried out.

Preferably, the device has at least one function selected from the group consisting of a lifestyle support function for persons with an advanced sleep phase, (sometimes referred to as "larks"), , a lifestyle support function for persons with a delayed sleep phase, (sometimes referred to as "owls"),, a lifestyle support function for persons with an irregular sleep-wake rhythm, a lifestyle support function for persons with a non-24-hour sleep-wake syndrome, (non-entrained type), or free-running disorder (FRD), a travelling function for persons travelling across time zones, a lifestyle support function for persons working late or night shifts, a lifestyle support function for persons suffering from seasonal affective disorder, (referred to as winter depression), and/or a lifestyle support function for persons suffering from, or being in danger of developing, obesity.

In order to support persons with an advanced sleep phase to shift their circadian phase and their sleeping times to later times of the day, the device is adapted in such way that it recommends that bright light should be sought in the evening before going to bed and bright light after wakeup should be avoided.

In order to support persons with a delayed sleep phase by shifting their circadian phase and their sleeping times to earlier times of the day, the device is adapted in such a way that it recommends that bright light should be avoided in the evening before going to bed and bright light after wakeup should be sought.

In order to support persons with irregular sleep-wake rhythm, the device is adapted in such a way that it recommends regular day-time exposure to bright light.

In order to support persons with non-24-hour sleep-wake syndrome (non-entrained type) or free-running disorder (FRD), the device is adapted in such a way that it recommends timed exposure to bright light in the morning.

For persons working late or night shifts, the device can be arranged in such way that personally recommended sleeping times, times in which bright light should be administered for improving alertness, and times in which bright light should be avoided in order to facilitate sleep during the day, are displayed. The calculation of such personal schedules is dependent on the personal shift schedules is done best using an application on a computer or web service and the results of that calculation being then entered or downloaded into the device.

For persons travelling across time zones, the device is adapted in such way that personally recommended times in which bright light should be administered and times in which bright light should be avoided in order to help the body to accommodate as quickly as possible to the new time zone are displayed. The calculation of such personal schedules is dependent on the direction (east/west or west/east), and on the number of travelled time zones, and is done best using an application on a computer or web service, while the results of that calculation being then entered or downloaded into the device.

For persons suffering from seasonal affective disorder (winter depression), the device is adapted to display recommendations with respect to different bright light therapy regimens. All regimens recommend bright light in the early morning, some regimens additionally recommend bright light in the evening after sunset. The duration of the bright light intervals also varies. The watch described in this invention allows the intuitive visualization of any treatment strategy that relies on regular timing of bright light exposure.

For persons suffering from, or being in danger of developing, obesity, the device is adapted to recommend, and motivate, the individual to increase his personal level of physical activity, in order to increase calorie consumption and increase mobility.

For persons suffering from, or being in danger of developing eating disorders, the device can be used to indicate wrong times of food intake and advise a change.

According to further aspects of the present invention, an activity visualization method as defined in claim 8 and a computer program as defined in claim 9 are presented.

### BRIEF DESCRIPTION OF THE DRAWINGS

In the following drawings:
Fig. 1 shows a first embodiment of an activity visualization device not forming part of the invention which has the shape of a wrist watch;
Fig. 2 shows a second embodiment of an activity visualization device according to the invention which has the shape of a wrist watch;
Figs. 3 - 8 show physical activities of six different individuals logged over a period of 13 days, and displayed in a linear fashion;
Fig. 9 shows two other embodiments of an activity visualization device not forming part of the invention which has the shape of a wrist watch.

### DETAILED DESCRIPTION OF THE DRAWINGS

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

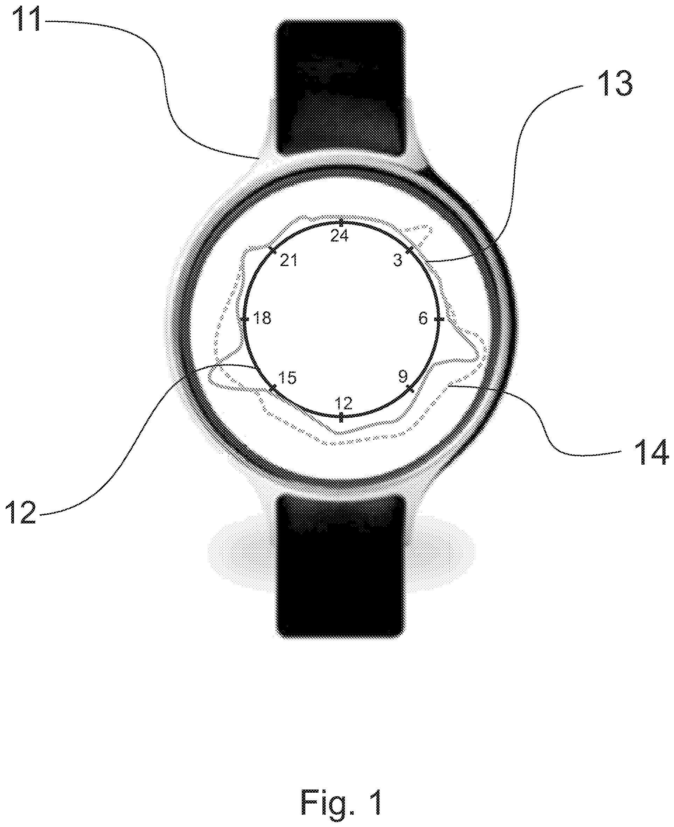

Fig. 1 shows schematically and exemplarily a first embodiment of an activity visualization device not forming part of the claimed invention which has the shape of a wrist watch 11. Said device comprises an accelerometer for measuring physical activity of an individual (not shown), an environmental light intensity sensor (not shown), a memory for logging said physical activity and light intensity over time (not shown), a processing unit for carrying out computational operations on the basis of the measured and/or logged data (not shown), and a visualization display for presentation of the measured and/or logged data and light intensity data, or data resulting from the computational operation thereof, said display being in the form of a circular dial 12 which covers a given period of time, i.e., in this case, a whole day period (24 hours distributed over 360°). The display furthermore displays a curved graph 13 (straight line) in which the physical activity of the individual carrying the device is displayed over time, as well as a curved graph 14 (dotted line) in which the environmental light intensity is displayed over time.

It can be seen, from the curves, that during the night both physical activity and environmental light intensity are at a minimum, with the exception of a light intensity peak at about 3.00 hrs, when the individual woke up, switched on the light, but stayed in bed. At about 6.30 hrs, the individual gets up, switches on the light and exerts some physical activity (morning sports). During the morning and noon, body activity is reduced while light intensity is high. At about 16.00 hrs, the individual is in a gym for doing a workout. After this, the individual goes home and has a rest. Daylight is slowly decreasing. At about 20.00 hrs, the individual prepares a meal, which results in short, slightly enhanced, physical activity. Later on, the individual goes to bed and switches off light.

In practice, different colours can be used for displaying the different curves, e.g., a yellow curve can be used for displaying the environmental light intensity, or a red curve can be used for displaying the physical activity. Likewise, other types of data can be displayed, e.g., as graphs in other colours, or as numerical values on a digital display. Examples for such data are physiological data of the individual, like heart rate, breathing rate, or body or skin temperature, or environmental data, like environmental temperature, environmental air humidity, environmental atmospheric pressure, environmental ozone concentration, sky cloudiness, sunrise and/or sunset data, and/or environmental light spectrum.

Furthermore, the device is capable of displaying activity data of multiple periods of time, e.g., multiple days. In this case, the most recent data set (e.g., the data set of the actual day) is displayed with highest colour saturation, while data sets of earlier periods (e.g., of the days before) are displayed with decreasing colour saturation. Thus, it is possible for the individual to see any trend change, e.g., in his physical activity, over time, in a time-resolved fashion. The individual can, e.g., compare the physical activity during his usual workout time from day to day.

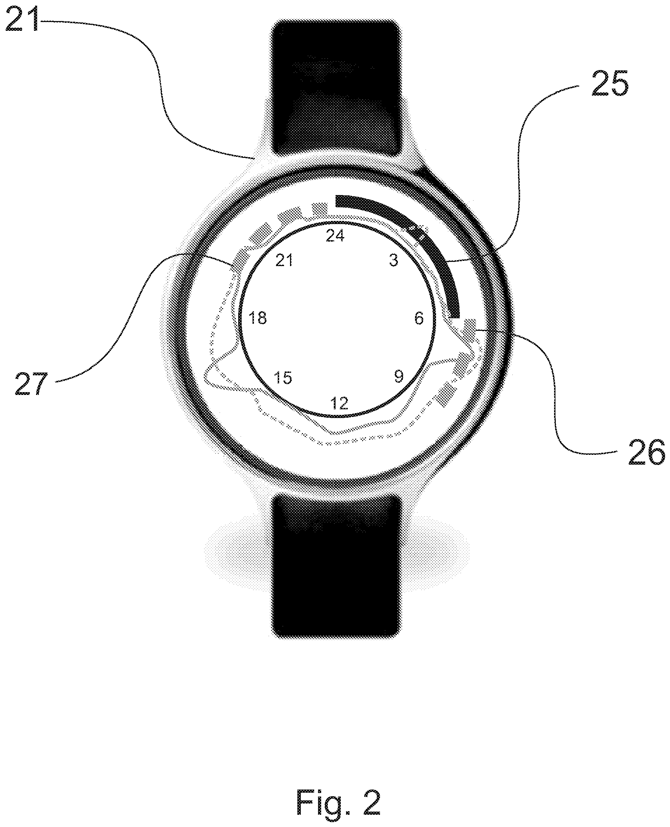

Fig. 2 shows schematically and exemplarily a second embodiment of an activity visualization device according to the invention which has the shape of a wrist watch 21. The shown device is furthermore adapted to display recommended values for the upper and/or lower limits of certain parameters, e.g., physical activity or environmental light intensity. The said recommendations are visualized by means of overlaid circular segments 25, 26, 27. Segment 25 indicates a recommended sleep time, in which exposure to light should be avoided in order to support a deep and restorative sleep. Segment 26 indicates a period of time in which the individual should expose himself or herself to a minimum level of environmental light intensity, in order to trigger his circadian clock. Segment 27 indicates a period of time in which the individual should avoid environmental light intensity exceeding a given limit, in order to prevent problems related to sleep. The device according to Fig. 2 can be used to implement a number of applications that improve the well-being of the individual, e.g., by targeting the improvement of sleep, the treatment of seasonal affective disorder, the treatment of circadian rhythm disorders, the improvement of physical or mental capabilities, etc. depending on which application is activated by a specific individual, and according to his personal preferences or physiological capabilities, different personalized limits and time schemes can be visualized on the display. Segments 25, 26, and 27, representing recommended sleep time, recommended period of time for exposure to a level of environmental light intensity, recommended period of time to avoid environmental light intensity exceeding a given limit, respectively, can be represented either by the positioning of the segments relative to the inner circle and to each other, or by assigning colors or gray levels to them, or a combination of positioning, colors, and levels of gray.

Basically, there are three different embodiments for setting personal recommended values: (i) the user can enter personal limit values and time schemes via implemented input facilities such as buttons or a touch screen; (ii) the device comprises processing capabilities for calculating light exposure limit values from the measured parameters (especially activity and light data); or (iii) the device comprises communication means for uploading measured parameters into a second device that performs calculation of recommended values and a means for receiving the calculated recommendations from that other device.

For the treatment of circadian rhythm sleep disorders the accurate timing of bright light exposure is of critical importance for the success of the therapy. For example, to treat a delayed sleep phase, the user typically has to seek bright light exposure in the early morning directly after wakeup and should avoid bright light in the evening before going to bed. The personally chosen time windows and their recommended value limits are visualized as circular segments so that the user can intuitively compare the real exposure values obtained within the measurement period with the recommended values.

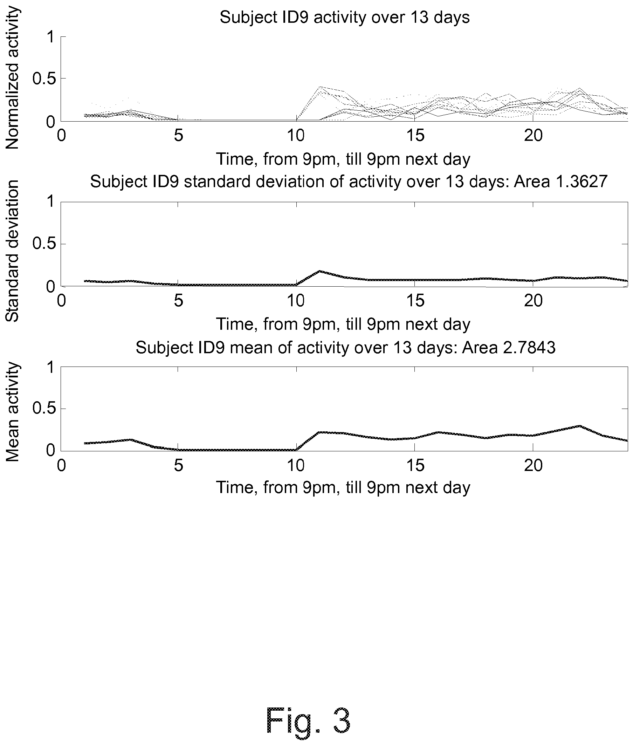

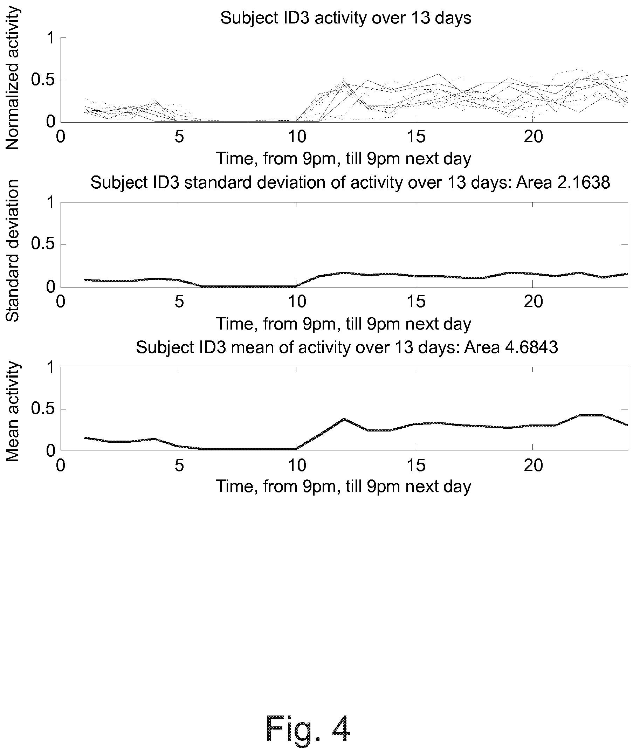

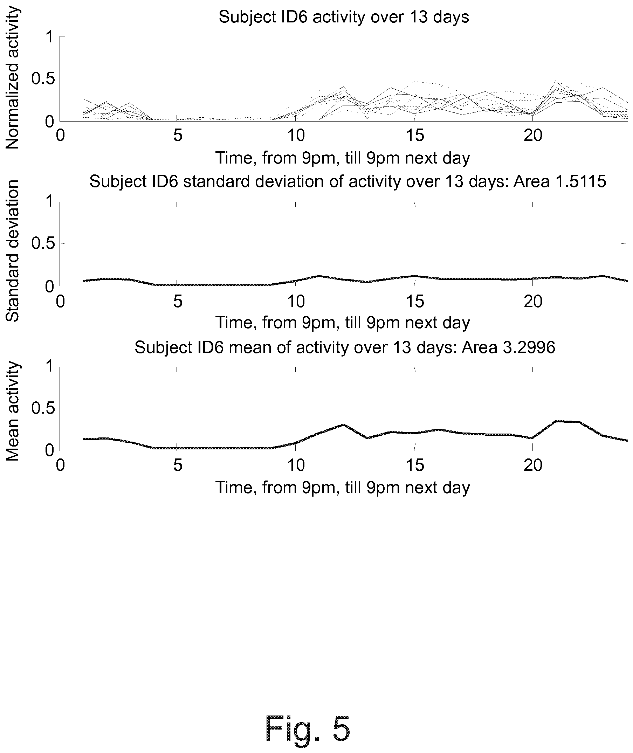

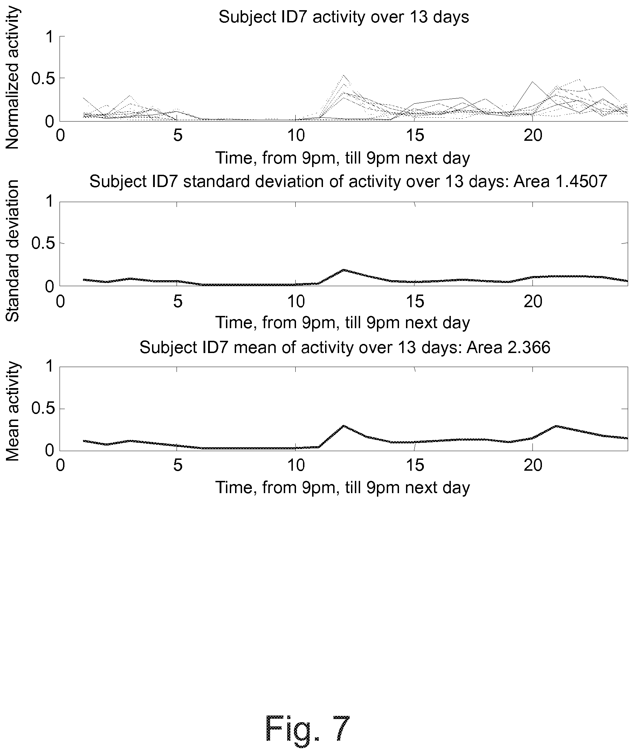

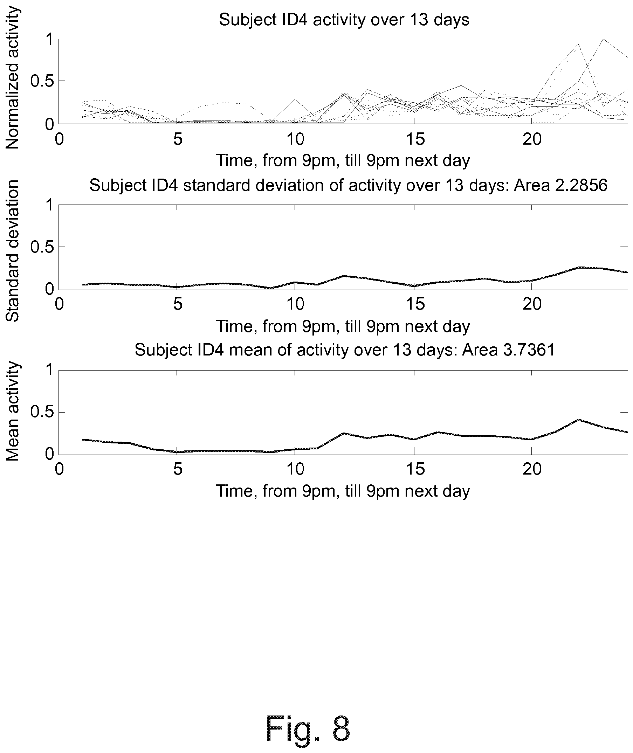

Figs. 3 - 8 show physical activities of five different individuals logged over a period of 13 days. Each figure has three sub-figures. The upper subfigure shows the normalized physical activity of a given individual (IDX) plotted over time. Different grey values indicate the data for different days. The lower subfigure shows the mean activity for all 13 days plotted over time, while the middle subfigure shows the respective standard deviations. See experimental section for details.

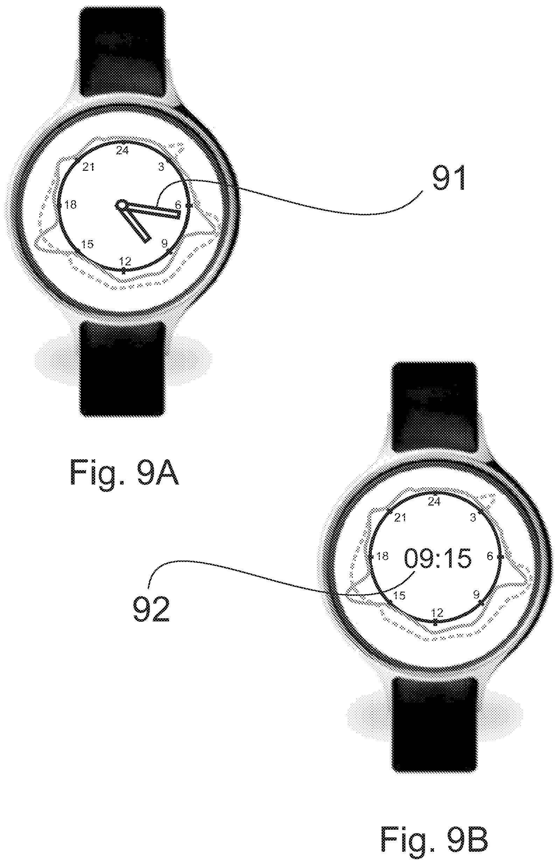

Fig. 9 shows two other embodiments of an activity visualization device not forming part of the invention which has the shape of a wrist watch. In Fig. 9a, the device comprises two hand-arrows 91 to indicate the actual time. In Fig. 9b, the device comprises a digital time display 92.

### EXPERIMENTS

In order to be certain about the feasibility of building the invention several validation tests were performed. Six male subjects of various ages were asked to continuously wear a device according to the invention comprising the said accelerometer for 2 weeks. Table 1 shows the different test subjects.

**Table 1**

| **ID No.** | **Fig. No.** | **age** |
|---|---|---|
| ID3 | 4 | 34 |
| ID6 | 5 | 49 |
| ID7 | 7 | 27 |
| ID9 | 3 | 38 |
| ID4 | 8 | 38 |
| ID2 | 6 | 29 |

For each subject, physical activity data were determined by means of the accelerometer, and logged in the device's memory. After the experiment, the data were transferred to a PC and analyzed by carrying out computational operations on the basis of the measured and/or logged data, i.e., normalization, calculation of mean activity and standard deviation. Furthermore, the data resulting from the computational operation were visualized by plotting them over time. Each plot displays activity of a subject during the first 13 days of the experiment. Each plot consists of three subplots: (1) normalized activity curves, (2) standard deviation of the mean activity curve over period of first 13 days and (3) mean activity curve over period of first 13 days. Results are shown in Figs 3 - 8.

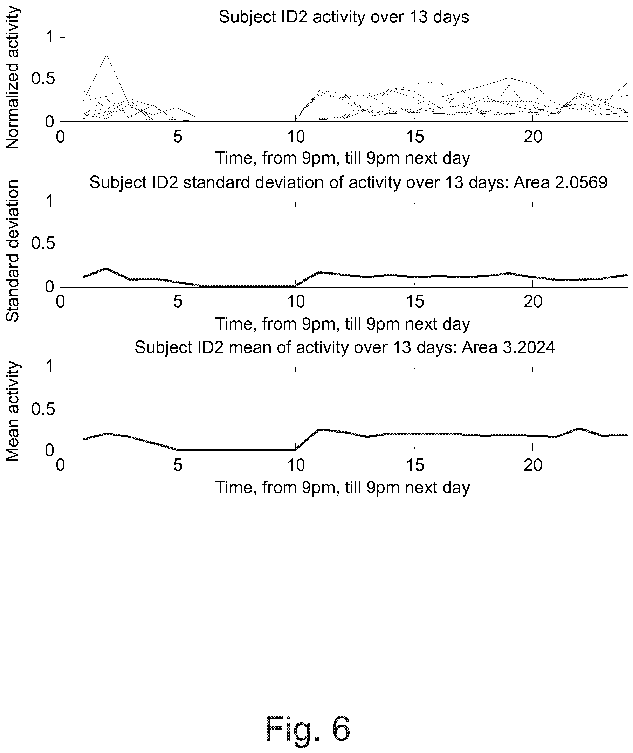

From the data visualized one can clearly distinguish people with steady lifestyle from those with a fuzzy one. Standard deviation curves of people with a fuzzy lifestyle are less flat and have sharp peak around less stable areas, e.g., during morning hours, see, e.g., Fig. 6. Also activity curves show steadiness of the pattern, thus for people with more steady lifestyle all the curves tend to overlap more.

## Claims

1. An activity visualization device for personal use, said device being adapted to be used in close physical contact with an individual, and said device at least comprising:
- an accelerometer configured to measure the physical activity of an individual,
- a memory configured to log said physical activity over time,
- a processing unit configured to carry out computational operations on the basis of measured and/or logged data, and
- a visualization display configured to present the measured and/or logged data, or data resulting from the computational operation thereof,
wherein said device adopts the shape of a watch, and
wherein said display is adapted in such way that it comprises a dial covering a given period of time, wherein physical activity data or data resulting from the computational operation thereof are displayed as a graph, or curve, over time,
**characterized in that** said device further comprises
- a communication means configured to obtain environmental data from an external source, said data selected from the group consisting of:
- environmental temperature,
- environmental air humidity,
- environmental atmospheric pressure,
- environmental ozone concentration,
- sky cloudiness,
- sunrise and/or sunset data,
- environmental light intensity, and/or
- environmental light spectrum, and
- an analysis unit configured to calculate recommendations for said individual with respect to physical activity and/or environmental light exposure based on the measured data and/or one or more of said obtained environmental data,
wherein said display is configured to display the calculated recommendations by means of overlaid circular segments on the dial, wherein said display is configured to display personally recommended levels of physical activity, to display personally recommended sleeping times, to display times when exposure to light should be avoided, or sought and/or to display times when food intake should be avoided, or carried out.

2. The activity visualization device according to claim 1, wherein said device adopts the shape of a wrist watch.

3. The activity visualization device according to claim 1-2, wherein said dial is a circular dial.

4. The activity visualization device according to any of claims 1 - 3, wherein said device further comprises at least one sensor for measuring physiological data of the individual, said sensor being selected from the group consisting of:
- heart rate sensor
- breathing rate sensor, and/or
- body or skin temperature sensor.

5. The activity visualization device according to any of claims 1 - 4, wherein said device further comprises at least one sensor for measuring environmental data, said sensor being selected from the group consisting of:
- environmental temperature sensor,
- environmental air humidity sensor,
- environmental atmospheric pressure sensor,
- environmental ozone concentration sensor,
- environmental noise sensor,
- environmental light intensity sensor, and/or
- environmental light spectrometer.

6. The activity visualization device according to any of claims 1 - 5, wherein said device further comprises a means for indicating the actual time and/or date.

7. The activity visualization device according to claim 1, wherein said device has at least one function selected from the group consisting of
- a lifestyle support function for persons with an advanced sleep phase;
- a lifestyle support function for persons with a delayed sleep phase;
- a lifestyle support function for persons with irregular sleep-wake rhythm;
- a lifestyle support function for persons with non-24-hour sleep-wake syndrome or free-running disorder, FRD;
- a travelling function for persons travelling across time zones;
- a lifestyle support function for persons working in late or night shifts; and/or
- a lifestyle support function for persons suffering from seasonal affective disorder.

8. A method for activity visualization by an activity visualization device for personal use comprising:
- measuring the physical activity of an individual,
- logging said physical activity over time on a memory,
- carrying out computational operations on the basis of measured and/or logged data by a processing unit, and
- presenting the measured and/or logged data, or data resulting from the computational operation thereof on a display which is adapted in such a way that it comprises a dial covering a given period of time,
wherein physical activity data or data resulting from the computational operation thereof are displayed as a graph, or curve, over time,
**characterized by**
- obtaining environmental data by a communication means from an external source, said data selected from the group consisting of:
- environmental temperature,
- environmental air humidity,
- environmental atmospheric pressure,
- environmental ozone concentration,
- sky cloudiness,
- sunrise and/or sunset data,
- environmental light intensity, and/or
- environmental light spectrum,
- calculating recommendations for said individual with respect to physical activity and/or environmental light exposure based on the measured data and/or one or more of said obtained environmental data, and
- displaying the calculated recommendations by means of overlaid circular segments, the recommendations being personally recommended levels of physical activity, personally recommended sleeping times, times when exposure to light should be avoided, or sought and/or times when food intake should be avoided, or carried out.

9. A computer program comprising program code means for causing a computer or processing unit to carry out the steps of the method as claimed in claim 8 when said computer program is executed on an activity visualization device according to claim 1.

## Patentansprüche

1. Vorrichtung für Aktivitätsvisualisierung für den persönlichen Gebrauch, wobei die Vorrichtung geeignet ist, um in engem physischem Kontakt mit einer Person verwendet zu werden, und wobei die Vorrichtung mindestens Folgendes umfasst:
- ein Beschleunigungsmesser, der konfiguriert ist, um die körperliche Aktivität einer Person zu messen,
- einen Speicher, der konfiguriert ist, um die körperliche Aktivität im Laufe der Zeit zu protokollieren,
- eine Verarbeitungseinheit, die konfiguriert ist, um Rechenoperationen auf der Grundlage von gemessenen und/oder protokollierten Daten durchzuführen, und
- eine Visualisierungsanzeige, die konfiguriert ist, um die gemessenen und/oder protokollierten Daten oder Daten, die sich aus deren Rechenoperation ergeben, darzustellen,
wobei die Vorrichtung die Form einer Uhr annimmt und
wobei die Anzeige so angepasst ist, dass sie ein Zifferblatt umfasst, das einen bestimmten Zeitraum abdeckt, wobei Daten der körperlichen Aktivität oder Daten, die sich aus ihrer Rechenoperation ergeben, als Graph oder Kurve im Laufe der Zeit angezeigt werden, **dadurch gekennzeichnet, dass** die Vorrichtung ferner umfasst
- ein Kommunikationsmittel, das konfiguriert ist, um Umgebungsdaten von einer externen Quelle zu erhalten, wobei die Daten ausgewählt sind aus der Gruppe bestehend aus:
- Umgebungstemperatur,
- Umgebungsluftfeuchtigkeit,
- Umgebungsluftdruck,
- Umgebungsozonkonzentration,
- Bewölkung des Himmels,
- Sonnenaufgangs- und/oder Sonnenuntergangsdaten,
- Umgebungslichtintensität und/oder
- Umgebungslichtspektrum und
- eine Analyseeinheit, die konfiguriert ist, um Empfehlungen für die Person in Bezug auf körperliche Aktivität und/oder Umgebungslichtexposition basierend auf den gemessenen Daten und/oder einem oder mehreren der erhaltenen Umgebungsdaten zu berechnen,
wobei die Anzeige konfiguriert ist, um die berechneten Empfehlungen mittels überlagerter Kreissegmente auf dem Zifferblatt anzuzeigen,
wobei die Anzeige konfiguriert ist, um persönlich empfohlene körperliche Aktivitätsniveaus anzuzeigen, um persönlich empfohlene Schlafzeiten anzuzeigen, um Zeiten anzuzeigen, in denen eine Lichtexposition vermieden oder gesucht werden sollte und/oder um Zeiten anzuzeigen, in denen eine Nahrungsaufnahme vermieden oder durchgeführt werden sollte.

2. Vorrichtung für Aktivitätsvisualisierung nach Anspruch 1, wobei die Vorrichtung die Form einer Armbanduhr annimmt.

3. Vorrichtung für Aktivitätsvisualisierung nach Anspruch 1 bis 2, wobei das Zifferblatt ein kreisförmiges Zifferblatt ist.

4. Vorrichtung für Aktivitätsvisualisierung nach einem der Ansprüche 1 bis 3, wobei die Vorrichtung ferner mindestens einen Sensor zum Messen physiologischer Daten der Person umfasst, wobei der Sensor ausgewählt ist aus der Gruppe bestehend aus:
- Herzfrequenzsensor
- Atemfrequenzsensor und/oder
- Körper- oder Hauttemperatursensor.

5. Vorrichtung für Aktivitätsvisualisierung nach einem der Ansprüche 1 bis 4, wobei die Vorrichtung ferner mindestens einen Sensor zum Messen von Umgebungsdaten umfasst, wobei der Sensor ausgewählt ist aus der Gruppe bestehend aus:
- Umgebungstemperatursensor,
- Umgebungsluftfeuchtigkeitssensor,
- Umgebungsluftdrucksensor,
- Umgebungsozonkonzentrationssensor,
- Umgebungsgeräuschsensor,
- Umgebungslichtintensitätssensor und/oder
- Umgebungslichtspektrometer.

6. Vorrichtung für Aktivitätsvisualisierung nach einem der Ansprüche 1 bis 5, wobei die Vorrichtung ferner ein Mittel zum Anzeigen der tatsächlichen Zeit und/oder des Datums umfasst.

7. Vorrichtung für Aktivitätsvisualisierung nach Anspruch 1, wobei die Vorrichtung mindestens eine Funktion aufweist, ausgewählt aus der Gruppe bestehend aus:
- eine Lebensstil-Unterstützungsfunktion für Personen mit einer fortgeschrittenen Schlafphase;
- eine Lebensstil-Unterstützungsfunktion für Personen mit einer verzögerten Schlafphase;
- eine Lebensstil-Unterstützungsfunktion für Personen mit unregelmäßigem Schlaf-Wach-Rhythmus;
- eine Lebensstil-Unterstützungsfunktion für Personen mit Nicht-24-Stunden-Schlaf-Wach-Syndrom oder Freilaufstörung, FRD;
- eine Reisefunktion für Personen, die durch Zeitzonen reisen;
- eine Lebensstil-Unterstützungsfunktion für Personen, die in Spät- oder Nachtschichten arbeiten; und/oder
- eine Lebensstil-Unterstützungsfunktion für Personen, die an einer saisonalen affektiven Störung leiden.

8. Verfahren zur Aktivitätsvisualisierung durch eine Vorrichtung für Aktivitätsvisualisierung für den persönlichen Gebrauch, umfassend:
- Messung der körperlichen Aktivität einer Person,
- Protokollieren der körperlichen Aktivität im Laufe der Zeit in einem Speicher,
- Durchführung von Rechenoperationen auf der Grundlage von gemessenen und/oder protokollierten Daten einer Verarbeitungseinheit, und
- Darstellen der gemessenen und/oder protokollierten Daten oder der Daten, die sich aus deren Rechenoperation ergeben, auf einem Display, das so angepasst ist, dass es ein Zifferblatt umfasst, das einen bestimmten Zeitraum abdeckt,
wobei Daten der körperlichen Aktivität oder Daten, die sich aus deren Rechenoperation ergeben, als Graph oder Kurve im Lauf der Zeit angezeigt werden,
**gekennzeichnet durch**
- Erhalten von Umgebungsdaten durch ein Kommunikationsmittel von einer externen Quelle, wobei die Daten ausgewählt sind aus der Gruppe bestehend aus:
- Umgebungstemperatur,
- Umgebungsluftfeuchtigkeit,
- Umgebungsluftdruck,
- Umgebungsozonkonzentration,
- Bewölkung des Himmels,
- Sonnenaufgangs- und/oder Sonnenuntergangsdaten,
- Umgebungslichtintensität und/oder
- Umgebungslichtspektrum,
- Berechnen von Empfehlungen für die Person in Bezug auf körperliche Aktivität und/oder Umgebungslichtexposition basierend auf den gemessenen Daten und/oder einem oder mehreren der erhaltenen Umgebungsdaten, und
- Anzeigen der berechneten Empfehlungen durch überlagerte Kreissegmente, wobei die Empfehlungen persönlich empfohlene körperliche Aktivität, persönlich empfohlene Schlafzeiten, Zeiten, in denen Lichtexposition vermieden oder gesucht werden sollte, und/oder Zeiten, in denen Nahrungsaufnahme vermieden oder durchgeführt werden sollte, sind.

9. Computerprogramm, das Programmcodemittel umfasst, um einen Computer oder eine Verarbeitungseinheit zu veranlassen, die Schritte des Verfahrens nach Anspruch 8 auszuführen, wenn das Computerprogramm auf einer Vorrichtung für Aktivitätsvisualisierung nach Anspruch 1 ausgeführt wird.

## Revendications

1. Dispositif de visualisation de l'activité à usage personnel, ledit dispositif étant adapté à être utilisé en contact physique étroit avec un individu, et ledit dispositif comprenant au moins:
- un accéléromètre configuré pour mesurer l'activité physique d'un individu,
- une mémoire configurée pour enregistrer ladite activité physique au cours du temps,
- une unité de traitement configurée pour exécuter des opérations de calcul sur la base de données mesurées et/ou enregistrées, et
- un afficheur de visualisation configuré pour présenter les données mesurées et/ou enregistrées, ou les données résultant de l'opération de calcul de celles-ci,
où ledit dispositif adopte la forme d'une montre, et
où ledit afficheur est adapté de telle manière qu'il comprend un cadran couvrant une période de temps donnée, où des données d'activité physique ou des données résultant de son opération de calcul sont affichées sous forme de graphique, ou courbe, au cours du temps,
**caractérisé en ce que** ledit dispositif comprend en outre
- un moyen de communication configuré pour obtenir des données environnementales d'une source externe, lesdites données étant choisies dans le groupe constitué par:
- la température ambiante,
- l'humidité de l'air ambiante,
- la pression atmosphérique ambiante,
- la concentration d'ozone dans l'environnement,
- la nébulosité du ciel,
- les données de lever du soleil et/ou coucher du soleil,
- l'intensité lumineuse ambiante, et/ou
- le spectre lumineux ambiant, et
- une unité d'analyse configurée pour calculer des recommandations pour ledit individu relativement à l'activité physique et/ou l'exposition à la lumière ambiante sur la base des données mesurées et/ou d'une ou plusieurs desdites données environnementales obtenues,
où ledit afficheur est configuré pour afficher les recommandations calculées au moyen de segments circulaires superposés sur le cadran,
où ledit afficheur est configuré pour afficher des niveaux d'activité physique recommandés personnellement, pour afficher les heures de sommeil recommandées personnellement, pour afficher les moments où l'exposition à la lumière doit être évitée, ou recherchée et/ou pour afficher les moments où la prise alimentaire doit être évitée, ou effectuée.

2. Dispositif de visualisation de l'activité selon la revendication 1, dans lequel ledit dispositif adopte la forme d'une montre-bracelet.

3. Dispositif de visualisation de l'activité selon les revendications 1 à 2, dans lequel ledit cadran est un cadran circulaire.

4. Dispositif de visualisation de l'activité selon l'une quelconque des revendications 1 à 3, dans lequel ledit dispositif comprend en outre au moins un capteur pour mesurer des données physiologiques de l'individu, ledit capteur étant sélectionné dans le groupe constitué par:
- un capteur de fréquence cardiaque
- un capteur de fréquence respiratoire, et/ou
- un capteur de température corporelle ou cutanée.

5. Dispositif de visualisation de l'activité selon l'une quelconque des revendications 1 à 4, dans lequel ledit dispositif comprend en outre au moins un capteur pour mesurer des données environnementales, ledit capteur étant sélectionné dans le groupe constitué par:
- un capteur de température ambiante,
- un capteur d'humidité de l'air ambiante,
- un capteur de pression atmosphérique ambiante,
- un capteur de concentration d'ozone dans l'environnement,
- un capteur de bruit ambiant,
- un capteur d'intensité lumineuse ambiante, et/ou
- un spectromètre de lumière ambiante.

6. Dispositif de visualisation de l'activité selon l'une quelconque des revendications 1 à 5, dans lequel ledit dispositif comprend en outre un moyen pour indiquer l'heure et/ou la date réelles.

7. Dispositif de visualisation de l'activité selon la revendication 1, dans lequel ledit dispositif a au moins une fonction sélectionnée dans le groupe constitué par
- une fonction de soutien au mode de vie pour les personnes en phase de sommeil avancé;
- une fonction de soutien au mode de vie pour les personnes en phase de sommeil retardé;
- une fonction de soutien au mode de vie pour les personnes ayant un rythme veille-sommeil irrégulier;
- une fonction de soutien au mode de vie pour les personnes ayant un syndrome veille-sommeil autre que 24 heures (syndrome hypernycthéméral) ou trouble free-running, FRD;
- une fonction de déplacement pour les personnes voyageant à travers les fuseaux horaires;
- une fonction de soutien au mode de vie pour les personnes travaillant tard ou de nuit; et/ou
- une fonction de soutien au mode de vie pour les personnes souffrant de troubles affectifs saisonniers.

8. Procédé de visualisation de l'activité par un dispositif de visualisation de l'activité à usage personnel consistant à:
- mesurer l'activité physique d'un individu,
- enregistrer ladite activité physique au cours du temps sur une mémoire,
- effectuer des opérations de calcul sur la base de données mesurées et/ou enregistrées par une unité de traitement, et
- présenter les données mesurées et/ou enregistrées, ou les données résultant de l'opération de calcul de celles-ci sur un afficheur qui est adapté de telle sorte qu'il comporte un cadran couvrant une période de temps donnée,
où les données de l'activité physique ou les données résultant de l'opération de calcul de celles-ci sont affichées sous forme de graphique, ou de courbe, au cours du temps,
**caractérisé par**
- l'obtention de données environnementales par un moyen de communication à partir d'une source externe, lesdites données étant sélectionnées dans le groupe constitué par:
- la température ambiante,
- l'humidité de l'air ambiante,
- la pression atmosphérique ambiante,
- la concentration d'ozone dans l'environnement,
- la nébulosité du ciel,
- les données de lever du soleil et/ou coucher du soleil,
- l'intensité lumineuse ambiante, et/ou
- le spectre lumineux ambiant,
- le calcul des recommandations pour ledit individu relativement à l'activité physique et/ou l'exposition à la lumière ambiante sur la base des données mesurées et/ou d'une ou plusieurs desdites données environnementales obtenues, et
- l'affichage des recommandations calculées au moyen de segments circulaires superposés, les recommandations étant des niveaux d'activité physique recommandés personnellement, les heures de sommeil recommandées personnellement, les moments où l'exposition à la lumière doit être évitée, ou recherchée et/ou les moments où la prise alimentaire doit être évitée, ou effectuée.

9. Programme informatique comprenant des moyens de code de programme pour amener un ordinateur ou une unité de traitement à réaliser les étapes du procédé selon la revendication 8 lorsque ledit programme informatique est exécuté sur un dispositif de visualisation de l'activité selon la revendication 1.
